# EUROPEAN PATENT APPLICATION

(11) **EP 4 159 834 A1**
(43) Date of publication of application: **05.04.2023**
(21) Application number: 21813327.0
(22) Date of filing: 27.05.2021
(51) Int. Cl.: C12M 1/00

(54) **SYSTEM FOR CULTURING CELL INCLUDING REMOVING AIR IN CELL CULTURING BAG**

(30) Priority: 28.05.2020 JP 2020093446
(71) Applicant: Toyo Seikan Group Holdings, Ltd., Shinagawa-ku Tokyo 141-8627 (JP)
(72) Inventor: SUENAGA Ryo, Yokohama-shi, Kanagawa 240-0062 (JP)
(74) Representative: Jones, Nicholas Andrew
(86) International application number: PCT/JP2021/020119
(87) International publication number: WO 2021/241665

(57) **Abstract**

An object of the present invention is to provide a novel approach capable of conveniently and efficiently removing air that has entered a cell culture bag at the time of addition of a culture medium. The present invention provides a system for culturing cells or cell aggregates using a cell culture bag, the cell culture bag having a lower face comprising a plurality of recesses, the culturing system comprising a mechanism which removes air in the cell culture bag through the cell culture bag by applying a pressure before cell addition to the cell culture bag supplemented with a culture medium.

## Description

### Technical Field

The present invention relates to a culturing system for removing air in a cell culture bag comprising a plurality of recesses, and producing cultured cells or cell aggregates in the bag.

### Background Art

In recent years, the mass culture of cells of interest in an artificial environment has been practiced in the fields of gene therapy, regenerative therapy, and the like. An exemplary approach involves using a cell culture plate with one or more recesses formed therein (also called "well plate"), introducing cells and a culture medium into each individual recess, and culturing the cells. However, this approach has the risk of contamination with foreign matter because the recesses are exposed to the atmosphere.

For example, larger containers or reduction in size and increase in the number of recesses have been required for obtaining large amounts of cells. A "micropattern plate" in which a plurality of small recesses are arranged within one well has been developed and used. However, in the micropattern plate, air often clogs the recesses when a medium is placed therein, due to the small recesses. In this case, in order to remove air, the air that clogs the recesses is taken off by directly blowing thereinto the flow of water caused using a pipette or the like. This makes the medium foamy so that cells may be difficult to see. Furthermore, the air that clogs the recesses is difficult to remove completely. Particularly, smaller holes and a larger number of recesses to be arranged (larger micropattern plate) make air more difficult to remove and place larger burdens on workers.

By contrast, an approach of performing mass culture in a closed manner using a gas permeable sac-like container (also referred to as a "cell culture bag") has been developed and reported (e.g., Patent Literature 1 to 4). This approach improves sealability as compared with the case of using the well plate mentioned above, and has the advantage that the risk of contamination with foreign matter can be reduced.

On the other hand, in closed culture using such a cell culture bag, air may also enter the bag at the time of addition of a culture medium and hinder the culture or observation of cells. Particularly, in the case of a cell culture bag comprising a plurality of very small recesses (e.g., Patent Literatures 3 and 4), air bubbles attached to the inside of the recesses may clog the recesses. This becomes large hindrance to cell culture.

The removal of air in a cell culture bag has heretofore been performed by physically stimulating the bag to float air bubbles attached to the inner wall of the bag into a culture medium, gathering the air bubbles to one location, and aspirating them with a syringe or the like. However, this procedure is troublesome and additionally has the difficulty in completely removing air. Particularly, the procedure is more difficult for a larger cell culture bag.

Hence, a novel approach capable of conveniently and efficiently removing air that has entered a cell culture bag at the time of addition of a culture medium, and performing cell culture has been desired in the art.

### Citation List

### Patent Literature

Patent Literature 1: Japanese Patent Laid-Open No. 2012-239401
Patent Literature 2: Japanese Patent Laid-Open No. 2016-86774
Patent Literature 3: WO2016/208526
Patent Literature 4: Japanese Patent Laid-Open No. 2019-118319

### Summary of Invention

### Technical Problem

Patent Literature 1 discloses that in using an incubator in a dry state, not in a usual humidified state, the partial pressure of an air component in a cell culture bag in which cells and a culture medium are enclosed is set to the same as or larger than the atmospheric pressure of dry air in the culture incubator, whereby the movement of air from the outside to the inside of the bag is prevented so that the generation of air bubbles in the cell culture bag can be prevented. This patent literature further states that even if air bubbles are still generated, the generated air bubbles can be eliminated by pressurizing the cell culture bag. The air targeted in Patent Literature 1 is not air that has entered the cell culture bag at the time of addition of a culture medium. In Patent Literature 1, the cell culture bag is sandwiched between a pressurization plate and a loading table and pressurized. In this respect, the surface of the bag is closely contacted with the surfaces of the pressurization plate and the loading table, and air bubbles generated in the bag cannot escape to the outside of the bag from the position closely contacted with the surfaces of the pressurization plate and the loading table so that the air bubbles are dissolved in a culture medium and disappear. Hence, air bubbles may reappear when the pressure on the cell culture bag was released.

Thus, none of the conventional techniques disclose or suggest by any means the complete removal of air that has entered a cell culture bag at the time of addition of a culture medium. Accordingly, an object of the present invention is to provide a novel approach capable of conveniently and efficiently removing air that has entered a cell culture bag at the time of addition of a culture medium.

### Solution to Problem

The present inventors have conducted diligent studies to attain the object and consequently found that a culture medium is added to a cell culture bag comprising a plurality of recesses, and a pressure is then applied to the bag, whereby air can be removed from the bag through the bag. The present invention is based on this novel finding and encompasses the following aspects.

[1] A system for culturing cells or cell aggregates using a cell culture bag,
   the cell culture bag having a lower face comprising a plurality of recesses,
   the culturing system comprising a mechanism which removes air in the cell culture bag through the cell culture bag by applying a pressure before cell addition to the cell culture bag supplemented with a culture medium.
[2] The culturing system according to [1], wherein the application of a pressure to the cell culture bag is performed by applying a pressure to the cell culture bag from above.
[3] The culturing system according to [1] or [2], further comprising a mechanism which stirs the cell culture bag supplemented with cells, followed by culture.
[4] The culturing system according to any of [1] to [3], further comprising a mechanism which recovers the formed cultured cells or cell aggregates after the completion of culture.
[5] The culturing system according to [4], further comprising a mechanism which recovers the formed cultured cells or cell aggregates by flipping the cell culture bag vertically after the completion of culture.
[6] The culturing system according to any of [1] to [5], wherein the lower face of the cell culture bag comprises 10 to 1000000 recesses.
[7] The culturing system according to any of [1] to [6], wherein the recesses in the cell culture bag have a spherical cap shape.
[8] The culturing system according to any of [1] to [7], wherein a diameter:depth ratio of the recesses in the cell culture bag is 1:0.25 to 1.
[9] The culturing system according to any of [1] to [8], wherein the recesses have a diameter of 300 µm to 1500 µm and a depth of 100 µm to 1000 µm.
[10] A method for removing air from a cell culture bag comprising a plurality of recesses, the method comprising the step of
   adding a culture medium to the cell culture bag, and then applying a pressure thereto to remove air in the cell culture bag through the cell culture bag.
[11] The method according to [10], wherein the lower face of the cell culture bag comprises 10 to 1000000 recesses.
[12] The method according to [10] or [11], wherein the recesses in the cell culture bag have a spherical cap shape.
[13] The method according to any of [10] to [12], wherein a depth:diameter ratio of the recesses in the cell culture bag is 1:1.5 to 2.5.
[14] The method according to any of [10] to [13], wherein a diameter:depth ratio of the recesses is 1:0.25 to 1.
[15] The method according to any of [10] to [14], wherein the recesses have a diameter of 300 µm to 1500 µm and a depth of 100 µm to 1000 µm.
[16] The method according to any of [10] to [15], wherein the application of a pressure to the cell culture bag is performed by applying a pressure to the cell culture bag from above.

The present specification encompasses the contents described in the specification and/or drawings of Japanese Patent Application No. 2020-93446 on which the priority of the present application is based.

All publications, patents and patent applications cited herein are incorporated herein by reference in their entirety.

### Advantageous Effects of Invention

The present invention can conveniently and efficiently remove air that has entered a cell culture bag comprising a plurality of recesses at the time of addition of a culture medium.

### Brief Description of Drawings

[Figure 1] Figure 1 is a schematic view showing one example of a cell culture bag comprising a plurality of recesses that can be used in the present invention.
Figure 1(a) is a perspective view, and Figure 1(b) is a transparent perspective view.
[Figure 2] Figure 2 is a schematic view showing another example of a cell culture bag that can be used in the present invention. Figure 2(a) is a perspective view, and Figure 2(b) is a transparent perspective view.
[Figure 3] Figure 3(1) is a perspective view showing one example of a placement table for placing a cell culture bag that can be used in the present invention. Figure 3(2) is a schematic view showing that recesses are formed on a lower face of the cell culture bag by placing the cell culture bag on the placement table, and applying a pressure to the cell culture bag sandwiched between the placement table and a pressing member.
[Figure 4] Figure 4 is a cross-sectional schematic view of a pressing apparatus that can be used in the present invention. Figure 4(a) shows an example having a placement face with formed openings to accept recesses of a cell culture bag, and Figure 4(b) shows an example having a flat placement face.
[Figure 5] Figure 5 is a perspective view showing another example of a pressing apparatus that can be used in the present invention.
[Figure 6] Figure 6 is a photographic view (A) and a graph (B) showing the relationship between the amplitude of an applied pressure and a preservation time as to the presence or absence of air bubbles in recesses.
[Figure 7-1] Figure 7-1 is a photographic view showing results of observing over time the presence or absence of air bubbles in recesses of a cell culture bag to which a pressure was applied.
[Figure 7-2] Figure 7-2 is a photographic view showing results of observing over time the presence or absence of air bubbles in recesses of a cell culture bag having a gas non-permeable film on a lower face to which a pressure was applied.
[Figure 8] Figure 8 is a photographic view showing results of observing over time the presence or absence of air bubbles in recesses of a large cell culture bag to which a pressure was applied.

### Description of Embodiments

### 1. Cell culture bag

As used herein, the "cell culture bag" means a culture container formed in a sac-like shape using a gas permeable flexible film material. The cell culture bag has a shape having at least a lower face comprising recesses, and preferably further has a shape having an upper face opposed to the lower face.

The "gas permeability" means a property of allowing a gas to pass through a flexible film material. In the cell culture bag according to the present invention, the transmission rate of oxygen measured at a test temperature of 37°C in Determination of Gas Transmission Rate of JIS K 7126 is preferably 5000 mL/(m²·day·atm) or more.

Examples of the flexible film material that can be used in the cell culture bag include, but are not limited to, films of resins such as polyethylene, polypropylene, ethylene-vinyl acetate copolymers, polyester, silicone elastomers, polystyrene elastomers, and tetrafluoroethylene-hexafluoropropylene copolymers (FEP). These films may each be used in the form of a singlelayer film or may be used in the form of a multilayer (e.g., two-layer or three-layer) film in which materials of the same type or different types are laminated. In the case of forming the cell culture bag by stacking and heat-sealing two films, it is preferred to have a layer that functions as a sealant layer in consideration of heat sealability. For example, the flexible film material can have a configuration of three layers, an inner layer, a base layer, and an outer layer, in order from the inner side of the cell culture bag. The base layer and the inner layer are preferably each constituted using a material having high gas permeability, heat sealability, and transparency. The inner layer is preferably constituted by a material having low cytotoxicity in addition to the characteristics described above. For example, a polyethylene resin such as linear low density polyethylene (LLDPE), very low density polyethylene (VLDPE) or ultra low density polyethylene (ULDPE), low density polyethylene (LDPE), or a blend thereof can be suitably used as such a material. A polyethylene resin having a density of 0.886 g/cm³ to 0.93 g/cm³ is suitable for the outer layer. The outer layer may be appropriately omitted. A surface serving as an inner face of the cell culture bag (at least the surface of the lower face) is preferably subjected to low cell adhesion treatment in order to facilitate gathering cells to the center of the bottom of the recesses. Examples of the low cell adhesion treatment include coating with phospholipid polymers, polyvinyl alcohol derivatives, surfactants, or albumin. The thickness of the film can be on the order of 50 to 300 **µ**m, preferably 80 to 160 **µ**m and may be the same thickness or different thicknesses between the upper face and the lower face of the cell culture bag.

The number of recesses carried by the lower face of the cell culture bag may be determined depending on the size of the cell culture bag or the desired number of cell aggregates, and can be appropriately selected from the range of, for example, 10 to 1000000.

The shape of the recesses is not particularly limited and is preferably a shape that facilitates gathering cells to the bottom of the recesses. Examples of the shape of the recesses include a spherical cap shape and a shape having a mortar-like (conical) depression. The diameter:depth ratio of the recesses is preferably 1:0.25 to 1, more preferably 1:0.3 to 0.6, in order to facilitate gathering cells to the bottom of the recesses. For example, in one embodiment, the opening size (diameter) thereof can be 300 **µ**m to 1500 **µ**m, and the depth can be 100 **µ**m to 1000 **µ**m**.**

The recesses are preferably arranged in a staggered pattern so as to maximize an area occupied by the recesses in the lower face, and may be arranged, if necessary, in a reticular pattern. The center-to-center spacing (pitch) between adjacent recesses 4 is not particularly limited and is preferably minimized within a range in which the outlines of adjacent recesses do not overlap each other. For example, the spacing between the outlines of the recesses can be set to larger than 0 to 1 mm, preferably 0.05 to 0.1 mm.

The thickness of the cell culture bag is not particularly limited and can be a thickness that enables a liquid depth (distance from the inner surface of a flat face (site except for the recesses) of the lower face film that comes in contact with a culture solution to the inner surface of the upper face film) to be on the order of 1 mm to 20 mm, preferably 2 mm to 8 mm, in supplementing with a culture medium.

The cell culture bag can have a tubular member allowing a medium, cells, and the like to flow (hereinafter, this member is also referred to as a "port"). The port can be formed from, for example, a thermoplastic resin such as polyethylene, polypropylene, vinyl chloride, a polyethylene elastomer, or a tetrafluoroethylene-hexafluoropropylene copolymer (FEP).

For the form of the "cell culture bag" according to the present invention, the forms of sac-like culture containers formed using conventional gas permeable flexible film materials known in the art (Japanese Patent No. 5344094, WO2016/208526, Japanese Patent Laid-Open No. 2016-86774, Japanese Patent Laid-Open No. 2019-118319, etc.) can be referred to.

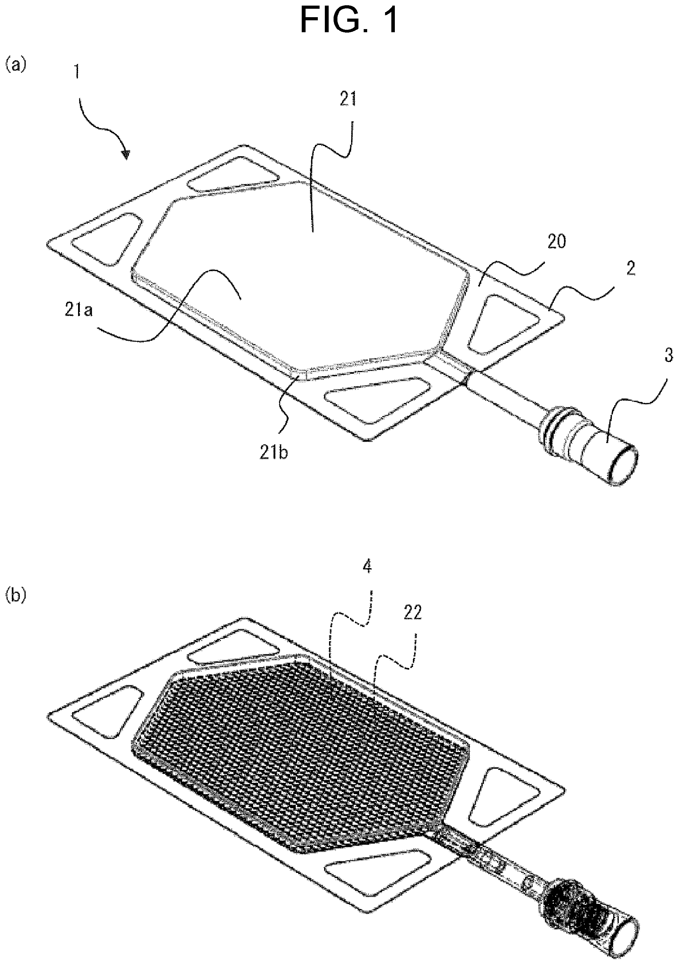

Figure 1 shows one example of the cell culture bag according to the present invention. A cell culture bag 1 has a sac-like configuration that comprises: a bag body 2 formed by stacking an upper face film 21 constituting the upper face and a lower face film 22 constituting the lower face, and sealing a peripheral part 20; and a port 3. A plurality of recesses 4 arranged in a staggered pattern are formed and retained by the lower face film 22 by a molding technique such as heat pressure molding. The upper face film 21 constituting the upper face has a bulging shape having a substantially flat top face portion 21a that covers over the entire plurality of recesses 4, and a sloping portion 21b around the top face portion 21a, and can thereby be sealed with the lower face film 22 to form the cell culture bag having a thickness. When the bag is filled with a medium or when a pressure is applied to the bag, deformation that lifts the periphery of the lower face film 22 can be suppressed.

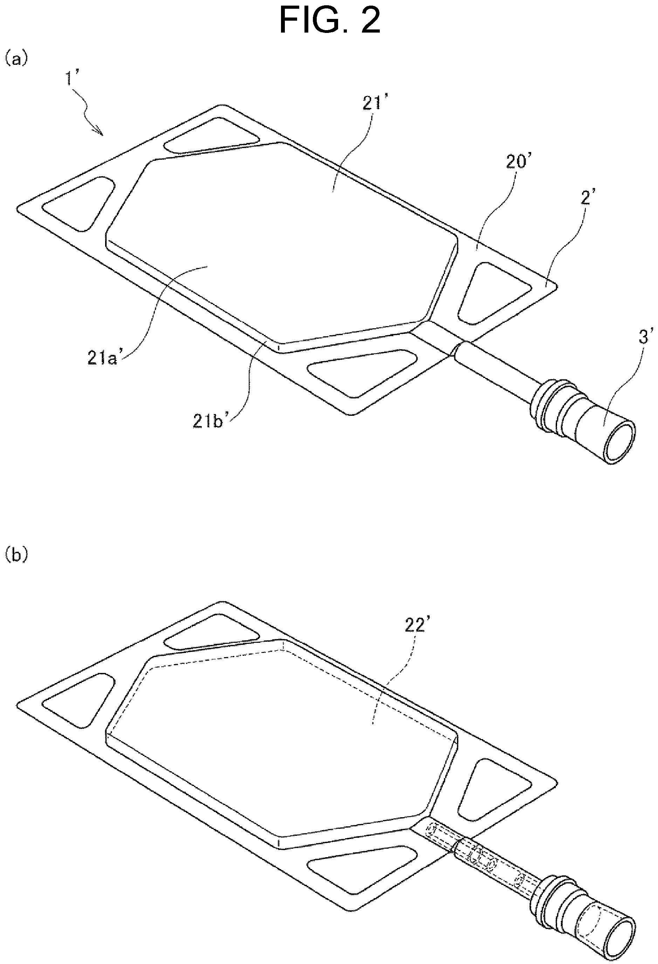

Figure 2 shows another example of the cell culture bag according to the present invention. In a cell culture bag 1', recesses are not directly formed in a lower face film 22' and are patterned after the shape of a placement face of a placement table contacted with the lower face film 22' following placement of the cell culture bag 1' on the placement table.

The placement table for the cell culture bag 1' is a plate-like member having a flat placement face for placing the cell culture bag, and depressions for forming recesses on the lower face of the cell culture bag. The placement table can be formed from a metal, a hard resin, or the like. The depressions of the placement face can have a shape capable of forming the recesses mentioned above on the lower face of the cell culture bag pressed thereagainst. The depressions may be formed by establishing corresponding recesses and/or projections on the placement face, or through-holes appropriately arranged in the placement face may be used as the depressions.

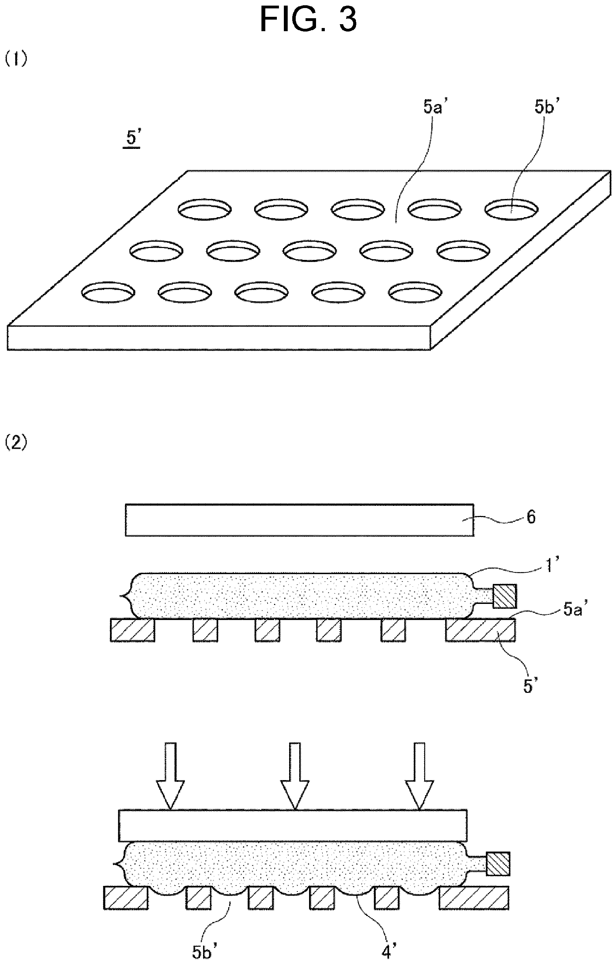

Figure 3(1) shows one example of a placement table 5' which forms recesses in the cell culture bag 1'. The placement table 5' has a flat placement face 5a' for placing the cell culture bag 1', and depressions 5b' in the form of through-holes for forming recesses on the lower face of the cell culture bag 1'. As shown in Figure 3(2), the cell culture bag 1' packed with cells and a predetermined culture solution is placed on the placement table 5'. Then, the cell culture bag 1' is sandwiched between a pressing member 6 mentioned later and the placement table 5', and a pressure is applied thereto so that the lower face film 22' is pressed against the placement face 5a and partially protruded in no contact with the placement face 5a from the portions of the depressions 5b' to form recesses 4'.

### 2. Pressing apparatus

In the present invention, a pressing apparatus having a configuration capable of applying a pressure to the cell culture bag packed with a predetermined culture medium can be used. The pressing apparatus has at least: a placement table on which the cell culture bag is to be placed; a pressing member which holds the upper face of the cell culture bag; a support mechanism which supports the up-and-down movement of one or both of the placement table and the pressing member; and a pressure application unit which presses one or both of the placement table and the pressing member against the cell culture bag, and may further has a liquid sending unit which injects or discharges cells or a medium via the port of the cell culture bag. In the case of applying a pressure to the cell culture bag by the self-weight of the pressing member, the pressure application unit may be omitted. Examples of the liquid sending unit include, but are not limited to, pumps such as peristaltic pumps, and syringes. The pressing apparatus may be portable so as to be transferred to an incubator for use, or may be installed in an incubator.

The placement table according to the present invention has a shape where its placement face supports the cell culture bag in low or no contact with the recesses. Unlike the cell culture bag of Patent Literature 1 mentioned above, this can maintain gas permeability without the close contact between the surface of the cell culture bag and the surface of the placement table upon application of a pressure, and enables air to move from the inside of the bag to the outside of the bag.

In the present invention, the phrase "applying a pressure" means that the internal pressure of the cell culture bag packed with a predetermined culture medium is increased. The application of a pressure can be performed by sandwiching the cell culture bag between the upper face (placement face) of the placement table and the lower face of the pressing member. Specifically, the pressing member can be pushed down toward the cell culture bag through the use of the support mechanism and the pressure application unit to apply a pressure to the cell culture bag from above, and/or the placement table can be pushed up toward the cell culture bag through the use of the support mechanism and the pressure application unit to apply a pressure to the cell culture bag from below.

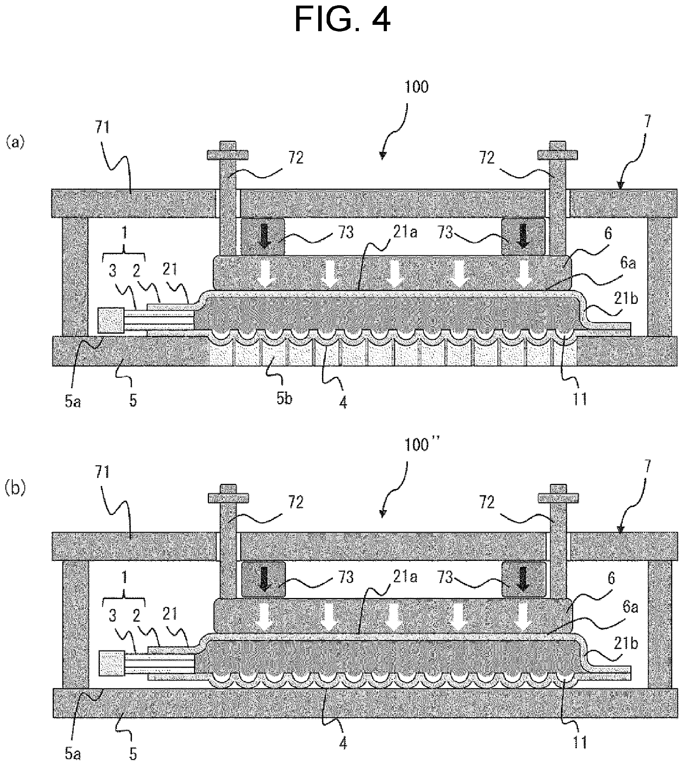

Figure 4 shows a cross-sectional schematic view of one example of a pressing apparatus that can be used in the present invention. A pressing apparatus 100 or 100'' of Figure 4 has a configuration that applies a pressure by pushing down a pressing member 6 from above toward a cell culture bag 1 placed on a placement table 5.

The pressing apparatus 100 or 100'' has: a placement table 5 on which the cell culture bag 1 is to be placed; a pressing member 6 having a bottom face 6a which presses a top face portion 21a of an upper face film 21; and a support mechanism 7 which supports the pressing member 6.

In Figure 4, the liquid sending unit which injects or discharges cells or a medium from a port 3 of the cell culture bag 1 is not shown.

The placement table 5 has a placement face 5a on which the cell culture bag 1 is horizontally placed. This placement face 5a can be a flat face (Figure 4(b)), or openings 5b which accept a plurality of recesses 4 formed in a lower face film 22 of the cell culture bag 1 may be formed (Figure 4(a)). In the presence of the openings 5b, the placement face 5a supports the lower face film 22 without coming into contact with the recesses 4. The shape of the openings 5b is not limited to such openings and may be, for example, recesses or may be a wire-mesh shape. Each opening 5b may have not only a shape that accepts each individual recess 4 but a shape capable of accepting a plurality of recesses 4.

The pressing member 6 is a plate-like member having a planar shape tailored to the top face portion 21a of the cell culture bag 1, and has a flat bottom face 6a.

The support mechanism 7 is constituted by: a frame 71 disposed on the placement face 5; guide pins 72 which extend upward from four corners of the upper face of the pressing member 6 and penetrate the frame 71 so as to be movable up and down; and pressure application units 73 which push down the pressing member 6. The pressure application units 73 apply a pressure to the cell culture bag 1 by pushing down the pressing member 6 toward the cell culture bag 1. The pressure to be applied can be adjusted by adjusting the force of the pressure application units 73 to push down the pressing member 6, and the distance travelled by the pressing member pushed down. The pressure application units 73 can have a configuration capable of pushing down the pressing member 6 by a predetermined force or at a predetermined distance, and can have a configuration made of screws, springs, magnets, or a combination thereof.

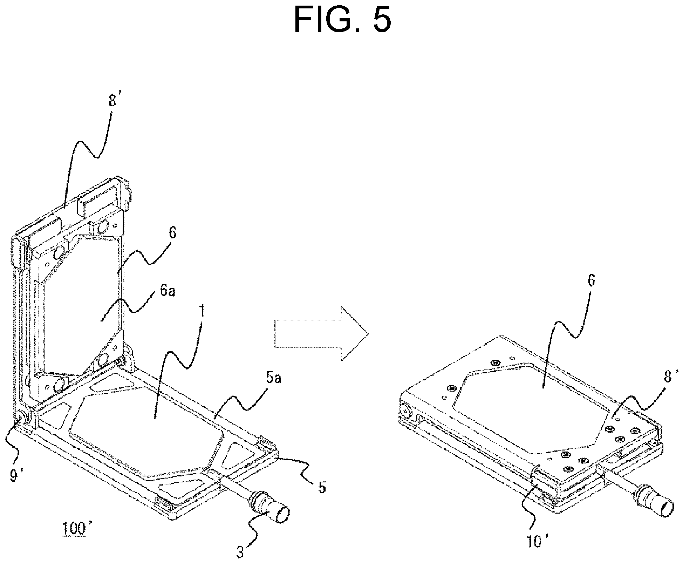

Figure 5 is a perspective view showing another example of a culture apparatus that can be used in the present invention. A culture apparatus 100' of Figure 5 has a placement table 5 having a placement face 5a, and a top cover 8' connected to this placement table 5 via a hinge 9'. The top cover 8' is configured so as to be openable and closable with the hinge 9' as an axis. The top cover 8' is constituted by: a pressing member 6 having a flat bottom face 6a tailored to a top face portion 21a of a cell culture bag 1; guide pins (not shown) which extend upward from four corners of the upper face of the pressing member 6 and penetrate the top cover 8' so as to be movable up and down; and pressure application units (not shown) which push down the pressing member 6 toward the cell culture bag 1.

When the cell culture bag 1 is housed in the culture apparatus 100', the top cover 8' is first opened via the hinge 9' (Figure 5(a)). In this state, the cell culture bag 1 is then placed on the placement face 5a having openings (not shown) that accept a plurality of recesses. The top cover 8' is then closed as shown in Figure 5(b) and maintained by lock mechanism 10'. A pressure can be applied to the cell culture bag 1 by the pressure application units which push down the pressing member 6 until a predetermined pressure is applied.

Both Figures 4 and 5 described above show examples using the cell culture bag 1. In the case of using a cell culture bag 1', a pressing apparatus having the same configuration as that of the pressing apparatus mentioned above can be used in each case except that a placement table having a placement face 5a' capable of forming recesses in the cell culture bag 1', such as a placement table 5', can be used as the placement table.

### 3. Cell

In the present invention, examples of the cells for use in the production of cell aggregates include, but are not particularly limited to, pluripotent stem cells and differentiation-induced cells thereof, neural stem cells, hepatocytes, corneal stem cells, pancreatic islet cells, cardiomyocytes.

In the present invention, the "pluripotent stem cells" refer to embryonic stem cells (ES cells) and cells potentially having pluripotent differentiation similar thereto, i.e., the ability to differentiate into various tissues (all the endoderm, the mesoderm, and the ectoderm) of a living body. Examples of the cells having pluripotent differentiation similar to that of the Es cells include "induced pluripotent stem cells" (in the present specification, also referred to as "iPS cells"). In the present invention, the pluripotent stem cells are preferably human pluripotent stem cells. The "induced pluripotent stem cells" refer to cells that are obtained by introducing mammalian somatic cells or undifferentiated stem cells with particular factors (nuclear reprogramming factors) such as Oct3/4, Sox2, Klf4 and c-Myc for reprogramming. The "differentiation-induced cells" of the pluripotent stem cells mentioned above mean cells that are obtained by inducing the differentiation of the pluripotent stem cells and characterized by a predetermined phenotype or the expression of a marker. The "marker" means a cell antigen specifically expressed from a predetermined cell type, or a gene thereof, such as "marker protein" or "marker gene".

The cells that can be used in the present invention may be cells collected from a living body, may be cultured cells, or may be frozen-thawed cells of these cells. The cells are used in a dissociated or dispersed state.

### 4. System for culturing cell or cell aggregate and method for removing air in cell culture bag packed with culture medium

### (1) Mechanism which removes air in cell culture bag

The system for culturing cells or cell aggregates according to the present invention comprises a mechanism which removes air in a cell culture bag, particularly, air bubbles attached to the inside of recesses, by applying a pressure to the bag packed with a culture medium. In the mechanism, the following is carried out.

The culture medium can be used by appropriately selecting a suitable one for the cells used. The culture medium is added from the port of the cell culture bag using a liquid sending unit such as a pump (e.g., a peristaltic pump) or a syringe. The amount of the culture medium added to the cell culture bag is appropriately adjustable depending on a factor such as the size of the cell culture bag, the number of cells added to the cell culture bag, a culture period, or the desired size of cell aggregates, and is not particularly limited. The bag is preferably filled up with the culture medium so as not to form an air layer in the cell culture bag.

The application of a pressure to the cell culture bag can be performed using the pressing apparatus mentioned above, and can be performed by sandwiching the cell culture bag between the placement table with the cell culture bag placed thereon and the pressing member that faces the top face portion of the cell culture bag.

The amplitude and application time of the pressure to be applied to the cell culture bag can be an amplitude and an application time that can remove air in the cell culture bag, particularly air bubbles attached to the inside of the recesses. The amplitude and the application time are appropriately adjustable depending on a factor such as the size of the cell culture bag, the size of openings of the recesses, or the depth of the recesses, and is not particularly limited. The cell culture bag can be treated with a pressure selected from the range of, for example, 0.001 kgf/cm² to 0.2 kgf/cm², preferably 0.01 kgf/cm² to 0.1 kgf/cm², for 1 hour to 3 days, preferably 2 hours to 1 day. In the present invention, the removal of air in the cell culture bag is carried out before addition of cells to the cell culture bag. This eliminates the need of taking into consideration the influence of an applied pressure to cells, and the optimum amplitude and application time of the pressure for removing air in the bag can be applied thereto. The application of the pressure to the cell culture bag may be performed inside an incubator for use in culture or outside the incubator.

According to the present invention, a pressure is applied to the cell culture bag, whereby air in the cell culture bag packed with a culture medium, particularly, air bubbles attached to the inside of the recesses, can be removed to the outside of the bag through a gas permeable flexible film material constituting the cell culture bag. This is different from an approach in which air or air bubbles are dissolved in a culture medium and disappear. Hence, in the case of releasing the applied pressure later, the removed air or air bubbles do not reappear.

### (2) Mechanism which cultures cell

The system for culturing cells or cell aggregates according to the present invention can comprise a mechanism which cultures cells in the cell culture bag packed with a culture medium from which air has been removed. In the mechanism, the following is carried out. The cells are added from the port of the cell culture bag using a liquid sending unit such as a pump (e.g., a peristaltic pump) or a syringe. The number of cells added to the cell culture bag is appropriately adjustable depending on a factor such as the size of the cell culture bag, a culture period, or the desired size of cell aggregates, and is not particularly limited. The number can be on the order of, for example, 1 × 10⁵ to 1 × 10⁷ cells/mL. The cells are added to the cell culture bag and then stirred with the culture medium. By such stirring, the numbers of cells that enter the individual recesses on the lower face of the cell culture bag can be substantially equalized in a subsequent step. The stirring may be performed manually or by a robot arm and/or can be performed using a vibrator.

The cells can be cultured under appropriate conditions for the cells used, and can be cultured in an incubator adjusted to an appropriate temperature (30 to 40°C, for example, 37°C), CO₂ concentration (5 to 10%, for example, 5%), and humidity (90 to 95%, for example, 95%). The culture period can be appropriately determined depending on a factor such as the desired size of cell aggregates or the type of the cells, and is not particularly limited. The culture period is, for example, 1 to 10 days, preferably 2 to 7 days.

### (3) Mechanism which recovers formed cultured cell or cell aggregate

The system for culturing cells or cell aggregates according to the present invention can comprise a mechanism which recovers cultured cells or cell aggregates formed in the recesses after the completion of culture. In the mechanism, the following is carried out. The cultured cells or the cell aggregates formed in the recesses can be recovered by use of an arbitrary approach capable of floating the cell aggregates in the culture medium and taking the cell aggregates out of the recesses. A physical approach can be used as such an approach. For example, an approach of flipping the cell culture bag vertically, applying vibration to the cell culture bag, thrusting up the recesses from the outer side of the cell culture bag using, for example, a protruding member (Japanese Patent Laid-Open No. 2019-118319), or removing the cell culture bag from the placement face and flattening the recesses (when the recesses are formed from the depressions of the placement face) can be used. Such an approach may be performed manually or by a robot arm and/or can be performed using a vibrator. Alternatively, air, a culture medium, or an appropriate buffer solution such as saline may be injected to the cell culture bag via the port of the cell culture bag, and the cell aggregates can be floated by the resulting jet flow and taken out of the recesses. The cell aggregates floated in the culture solution can be recovered, together with the culture medium, etc., from the port of the cell culture bag.

Hereinafter, the present invention will be described with reference to Examples. However, the present invention is not limited by these Examples.

### Examples

### Experiment 1. Removal of air in medium-sized cell culture bag

### (1-1) Medium-sized cell culture bag

A medium-sized cell culture bag having the same configuration as that of the cell culture bag shown in Figure 1 was prepared and used. Specifically, the cell culture bag had a configuration that was prepared by stacking two gas permeable flexible polyethylene films each having a thickness of 100 **µ**m, and sealing their peripheries (area inside the seal: 50 cm²), and had a port. The lower face film of the cell culture bag had 1.8 × 10⁴ recesses each having a diameter of 500 **µ**m and a depth of 200 **µ**m, and the upper face film thereof had a bulging shape having a 4 mm high bulge. The inner faces of the films were subjected to low cell adhesion coating with a phospholipid polymer.

### (1-2) Experimental method

10 mL of a culture medium was added from the port of the medium-sized cell culture bag, and the port was closed. While each pressure of 0 kgf, 0.5 kgf, 1.0 kgf, 2.0 kgf, or 4.0 kgf (surface pressure: 0 kgf/cm², 0.01 kgf/cm², 0.02 kgf/cm², 0.04 kgf/cm², or 0.08 kgf/cm²) was applied thereto using a pressing apparatus, the bag was preserved in a CO₂ incubator (37°C, 95% humidity, 5% CO₂).

The presence or absence of air bubbles in the recesses was observed before preservation (0 hours) and 2.5 hours, 4.5 hours, 9 hours, 15 hours, and 72 hours after preservation.

### (1-3) Results

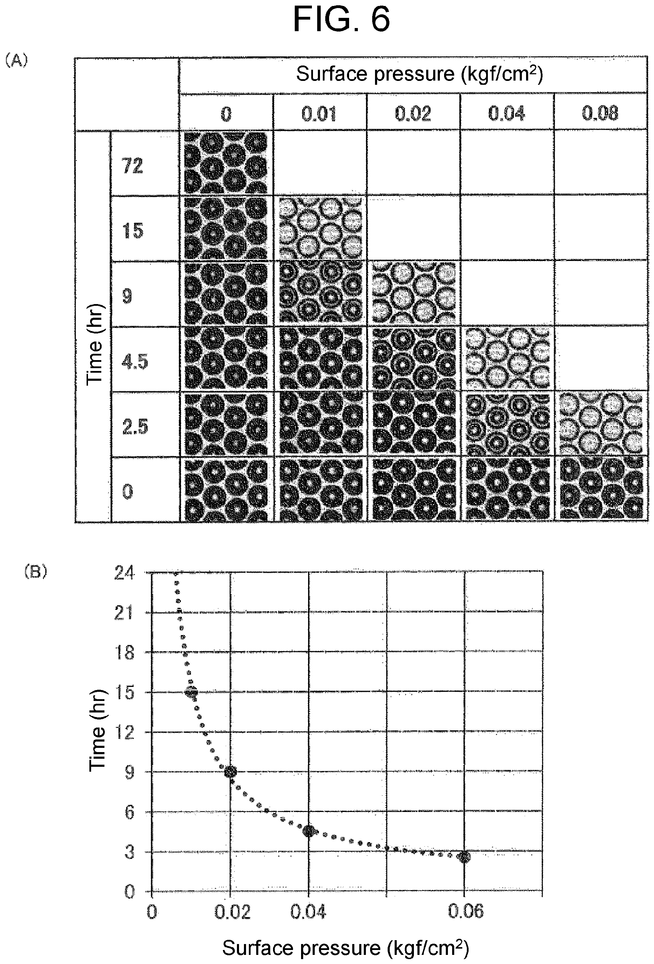

Figure 6 shows the relationship between the amplitude of an applied pressure and a preservation time as to the presence or absence of air bubbles in the recesses. In the photographic view (A) of Figure 6, the black inside of the recesses (sites that look round) indicates that air bubbles are present, whereas the gray-white inside thereof indicates that air bubbles are absent. The graph (B) of Figure 6 shows the relationship between the amplitude of the applied pressure and the time required to eliminate air bubbles in the recesses on the basis of each box of the photographic view. A shorter time required to eliminate air bubbles was observed with increase in applied pressure. For example, in the case of eliminating air bubbles in the recesses overnight (approximately 12 hours) as to the medium-sized cell culture bag, an applied pressure of 0.5 kgf to 1.0 kgf was confirmed to suffice. As the recess diameter is increased, the volume of air present therein is increased. Therefore, the recess diameter is also capable of serving as a factor that influences the time required to eliminate air bubbles. In the case of changing the recess diameter from 0.5 **µ**m to 1.26 **µ**m in the medium-sized cell culture bag mentioned above, the time required to eliminate air bubbles was extended to 57 hours from 9 hours.

### Experiment 2. Removal of air in cell culture bag having gas non-permeable film on lower face

### (2-1) Medium-sized cell culture bag having gas non-permeable film on lower face

A medium-sized cell culture bag having the same configuration as that of the cell culture bag used in the experiment 1 was prepared and used except that the gas permeable film on the lower face (recess side) was changed to a multilayer gas non-permeable film prepared through the adhesion between polyethylene and an ethylene-vinyl alcohol copolymer (EVOH). The gas non-permeable film (thickness: 118.5 **µ**m) consisted of polyethylene (thickness: 67.7 **µ**m), an adhesive layer (thickness: 17.3 **µ**m), and EVOH (thickness: 30.5 **µ**m) in order from the inside. The gas transmission rates of the gas permeable film and the gas non-permeable film used in the experiments will be given below.

**[Table 1]**

| Gas transmission rate of each film (37°C-dry) | | |
|---|---|---|
| | Oxygen (mL/m²·day atm) | Nitrogen (mL/m²·day atm) |
| Gas permeable film | 8250 | 3040 |
| Gas non-permeable film | 91.7 | 34.5 |

### (2-2) Experimental method

10 mL of a culture medium was added from the port of each of the cell culture bag having a gas non-permeable film on the lower face (hereinafter, referred to as the "gas non-permeable cell culture bag") and the gas permeable cell culture bag prepared in the experiment 1, and the port was closed. While a pressure of 0.8 kgf (surface pressure: 0.016 kgf/cm²) was applied to each bag using a pressing apparatus, the bag was preserved in a CO₂ incubator (37°C, 95% humidity, 5% CO₂).

The presence or absence of air bubbles in the recesses was observed before preservation (0 hours) and 1 hours to 23 hours after preservation.

### (2-3) Results

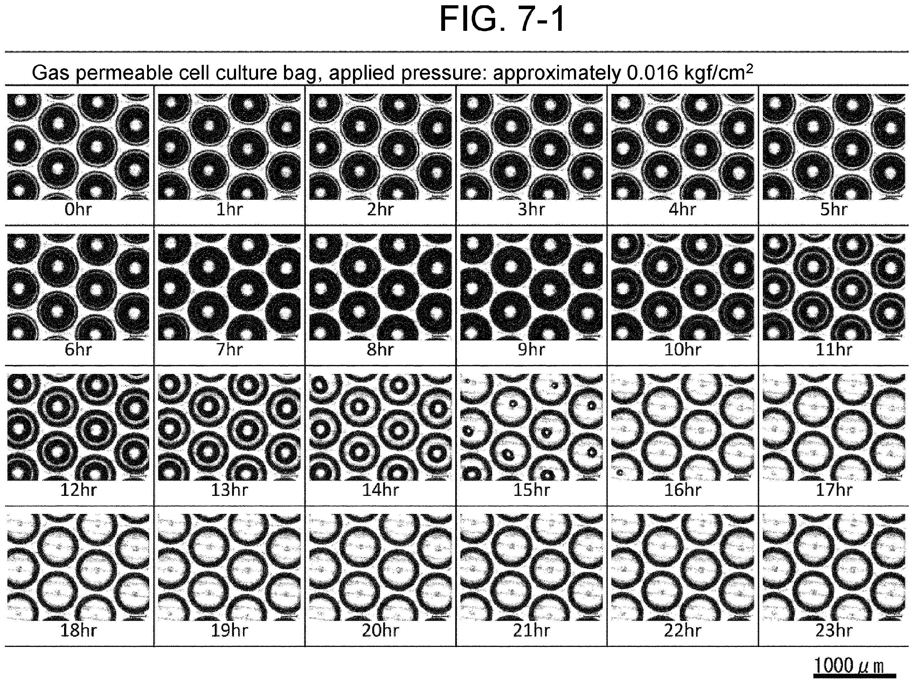

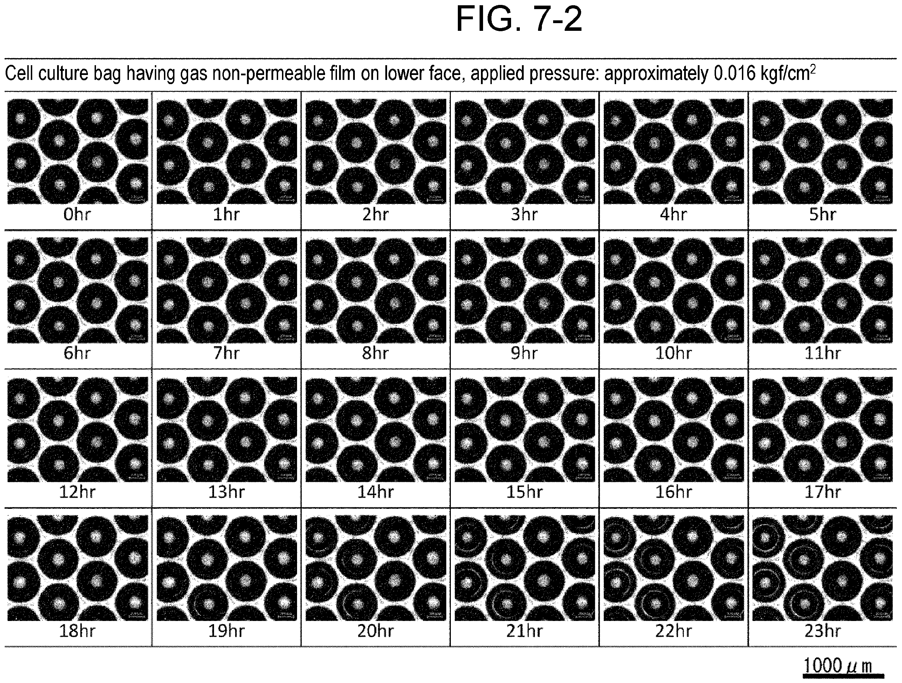

Figure 7-1 (gas permeable cell culture bag) and Figure 7-2 (gas non-permeable cell culture bag) show results of observing over time the presence or absence of air bubbles in the recesses of each cell culture bag. In the photographic view of each figure, the black inside of the recesses (sites that look round) indicates that air bubbles are present, whereas the gray-white inside thereof indicates that air bubbles are absent.

In the gas permeable cell culture bag (Figure 7-1), air bubbles were eliminated 15 to 16 hours after preservation. By contrast, in the gas non-permeable cell culture bag (Figure 7-2), air bubbles were not eliminated even after a lapse of 23 hours after preservation. From these results, the present approach was confirmed to remove air bubbles present in the recesses of the cell culture bag through a gas permeable film.

### Experiment 3. Removal of air in large cell culture bag (3-1) Large cell culture bag

A large cell culture bag having the same configuration as that of the cell culture bag shown in Figure 1 was prepared and used. The cell culture bag had a configuration that was prepared by stacking two gas permeable flexible polyethylene films each having a thickness of 100 **µ**m, and sealing their peripheries (area inside the seal: 1,000 cm²), and had a port. The lower face film of the cell culture bag had 65 × 10⁴ recesses each having a diameter of 350 **µ**m and a depth of 150 **µ**m, and the upper face film thereof had a bulging shape having a 4 mm high bulge. The inner faces of the films were subjected to low cell adhesion coating with a phospholipid polymer.

### (3-2) Experimental method

200 mL of a culture medium was added from the port of the large cell culture bag, and the port was closed. While each pressure of 1.0 kgf (surface pressure: 0.01 kgf/cm²) was applied thereto using a pressing apparatus, the bag was preserved in a CO₂ incubator (37°C, 95% humidity, 5% CO₂).

### (3-3) Results

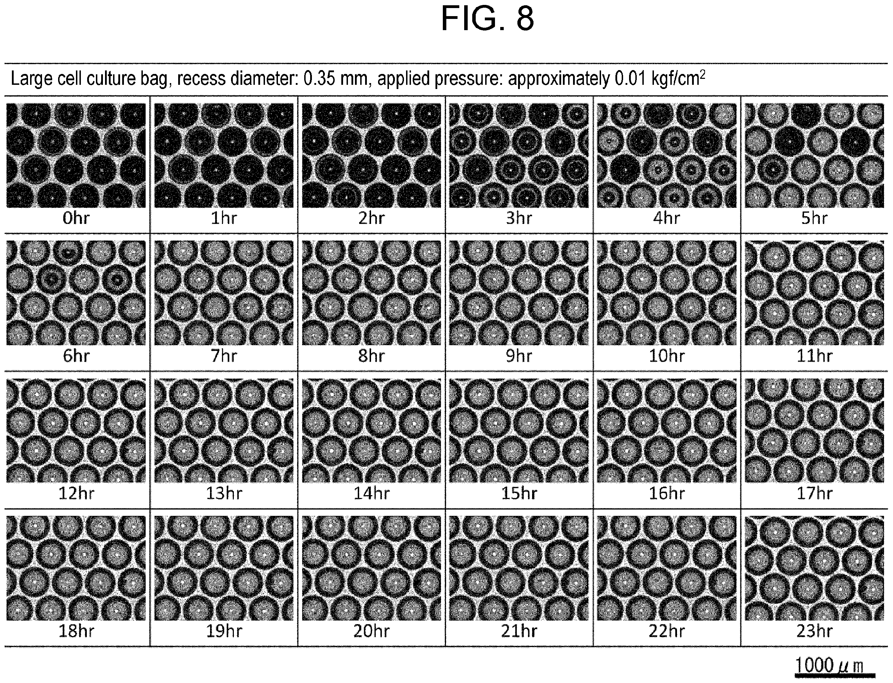

Figure 8 shows results of observing over time the presence or absence of air bubbles in the recesses of the large cell culture bag. In the photographic view of Figure 8, the black inside of the recesses (sites that look round) indicates that air bubbles are present, whereas the gray-white inside thereof indicates that air bubbles are absent. Air bubbles in the recesses were eliminated approximately 8 hours after preservation. Even for the large cell culture bag, it was confirmed that air bubbles in the recesses can be removed by the application of a pressure.

From the results described above, the method of the present invention was confirmed to be able to conveniently and efficiently remove air bubbles attached to the inside of a plurality of recesses in a cell culture bag packed with a culture medium. The diameter (350 **µ**m) of the recesses in the large cell culture bag used in this experiment was smaller than the diameter (500 **µ**m) of the recesses in the medium-sized cell culture bag mentioned above, and the size of air bubbles in the recesses was also smaller. Therefore, these air bubbles were removed in a short time.

### Reference Signs List

1 and 1': Cell culture bag
2 and 2': Bag body
20 and 20': Peripheral part
21 and 21': Upper face film
21a and 21a': Top face portion
21b and 21b': Sloping portion
22 and 22': Lower face film
3 and 3': Port
4 and 4' Recess
5 and 5': Placement table
5a and 5a': Placement face
5b: Opening
5b': Depression
6: Pressing member
6a: Bottom face
7: Support mechanism
71: Frame
72: Guide pin
73: Pressure application unit
8': Top cover
9': Hinge
10': Lock mechanism
100, 100', and 100'': Pressing apparatus
11: Air

## Claims

1. A system for culturing cells or cell aggregates using a cell culture bag,
the cell culture bag having a lower face comprising a plurality of recesses,
the culturing system comprising a mechanism which removes air in the cell culture bag through the cell culture bag by applying a pressure before cell addition to the cell culture bag supplemented with a culture medium.

2. The culturing system according to claim 1, wherein the application of a pressure to the cell culture bag is performed by applying a pressure to the cell culture bag from above.

3. The culturing system according to claim 1 or 2, further comprising a mechanism which stirs the cell culture bag supplemented with cells, followed by culture.

4. The culturing system according to any one of claims 1 to 3, further comprising a mechanism which recovers the formed cultured cells or cell aggregates after the completion of culture.

5. The culturing system according to claim 4, further comprising a mechanism which recovers the formed cultured cells or cell aggregates by flipping the cell culture bag vertically after the completion of culture.

6. The culturing system according to any one of claims 1 to 5, wherein the lower face of the cell culture bag comprises 10 to 1000000 recesses.

7. The culturing system according to any one of claims 1 to 6, wherein the recesses in the cell culture bag have a spherical cap shape.

8. The culturing system according to any one of claims 1 to 7, wherein a diameter:depth ratio of the recesses in the cell culture bag is 1:0.25 to 1.

9. The culturing system according to any one of claims 1 to 8, wherein the recesses have a diameter of 300 **µ**m to 1500 **µ**m and a depth of 100 **µ**m to 1000 **µ**m.

10. A method for removing air from a cell culture bag comprising a plurality of recesses, the method comprising the step of
adding a culture medium to the cell culture bag, and then applying a pressure thereto to remove air in the cell culture bag through the cell culture bag.

11. The method according to claim 10, wherein the lower face of the cell culture bag comprises 10 to 1000000 recesses.

12. The method according to claim 10 or 11, wherein the recesses in the cell culture bag have a spherical cap shape.

13. The method according to any one of claims 10 to 12, wherein a depth:diameter ratio of the recesses in the cell culture bag is 1:1.5 to 2.5.

14. The method according to any one of claims 10 to 13, wherein a diameter:depth ratio of the recesses is 1:0.25 to 1.

15. The method according to any one of claims 10 to 14, wherein the recesses have a diameter of 300 **µ**m to 1500 **µ**m and a depth of 100 **µ**m to 1000 **µ**m.

16. The method according to any one of claims 10 to 15, wherein the application of a pressure to the cell culture bag is performed by applying a pressure to the cell culture bag from above.
